# EUROPEAN PATENT SPECIFICATION

(11) **EP 2 862 567 B1**
(45) Date of publication and mention of the grant of the patent: **26.07.2017**
(21) Application number: 13742149.1
(22) Date of filing: 17.06.2013
(51) Int. Cl.: A61K 9/14, A61K 36/889

(54) **COMPOUNDS FROM THE FRUITS OF ACROCOMIA CRISPA AND ACROCOMIA ACULATA AGAINST INFLAMMATION AND OXIDATIVE STRESS**
VERBINDUNGEN AUS FRÜCHTEN DER ACROCOMIA CRISPA UND ACROCOMIA ACULATA GEGEN ENTZÜNDUNGEN UND OXIDATIVEN STRESS
COMPOSÉES DES FRUITS DE ACROCOMIA CRISPA ET ACROCOMIA ACULATA CONTRE L'INFLAMMATIONS ET STRESS OXIDATIF

(30) Priority: 19.06.2012 CU 9712
(43) Date of publication of application: 22.04.2015
(73) Proprietor: Centro Nacional De Investigaciones Cientificas (CNIC), Ciudad de la Habana 12100 (CU)
(72) Inventor: GONZALES CANAVACIOLO, Victor Luis, 11600 La Habana (CU); SIERRA PÉREZ, Roxana, de la Caridad, La Lisa 13600 La Habana (CU); MAS FERREIRO, Rosa María, 11600 La Habana (CU); PÉREZ GUERRA, Yohani, La Lisa 13600 La Habana (CU); OYARZÁBAL YERA, Ámbar, Boyeros 19280 La Habana (CU); RODRÍGUEZ LEYES, Eduardo Antonio, La Lisa 13600 La Habana (CU); MOLINA CUEVAS, Vivían, 11600 La Habana (CU); GÁMEZ MENÉNDEZ, Rafael, 11600 La Habana (CU)
(74) Representative: Carpintero Lopez, Francisco
(86) International application number: PCT/CU2013/000003
(87) International publication number: WO 2013/189467

(56) References cited:
- WO-A1-2006/078848
- GB-A- 191 412 621
- MICHELLE CARDOSO COIMBRA ET AL: "Characterization of the Pulp and Kernel Oils from Syagrus oleracea, Syagrus romanzoffiana, and Acrocomia aculeata", JOURNAL OF FOOD SCIENCE, vol. 76, no. 8, 17 October 2011 (2011-10-17), pages C1156-C1161, XP055079142, ISSN: 0022-1147, DOI: 10.1111/j.1750-3841.2011.02358.x
- COLLIN: "The kernel-fats of some members of the Palmae: Acrocomia sclerocarpa Mart. (Gru-gru palm), Manicaria saccifera Gaertn., Astrocaryum Tucuma Mart., Maximiliana caribaea Griseb., Attalea excelsa Mart. (Pallia palm), and Cocos nucifera Linn. (coconut).", BIOCHEMICAL JOURNAL, vol. 27, no. 5, 1 January 1933 (1933-01-01), pages 1366-1372, XP55094135, ISSN: 0264-6021
- HERNANDEZ GALICIA ET AL: "Studies on hypoglycemic activity of Mexican medicinal plants.", PROCEEDINGS OF THE WESTERN PHARMACOLOGY SOCIETY, vol. 45, 1 January 2002 (2002-01-01), pages 118-124, XP55094148, ISSN: 0083-8969

## Description

### Technical Section

This invention is related to the obtainment of a new active ingredient that can be used as nutritional supplement, pharmaceutical or cosmetic product for preventing and/or treating the inflammatory processes and oxidative stress that accompany the ageing process and many pathologies, like the benign prostatic hyperplasia (BPH), prostatitis, osteoarthritis, among others; and also to the process for obtaining such active ingredient from green or ripe fruits of both *Acrocomia crispa* and *Acrocomia aculeata,* species of Arecaceae family. This active ingredient includes a mixture of linear fatty acids from 6 to 28 carbon atoms, mainly the saturated: C_{8:0}, C_{10:0}, C_{12:0}, C_{14:0}, C_{16:0} and C_{18:0}, and the unsaturated: C_{16:1}, C_{18:1}, C_{18:2} and C_{18:3}, and could also contain sterols and high molecular weight fatty alcohols.

The obtainment process of this active ingredient starts by drying and milling the fruits, and may include a partial or total hydrolysis (enzymatic, basic or acid) of the vegetable material or of its lipid fraction, with a further recovery of the fatty acids; the selective extraction of the lipid fraction can be done by organic solvents or by extraction with supercritical fluids. Both procedures, with and without hydrolysis, allow obtain active ingredients with similar pharmacological and physiological effects.

The present invention is related with the food and pharmaceutical industries, since the referred active ingredient can be used as such or well in doses between 50 and 1000 mg as part of nutritional, cosmetic and/or pharmaceutical formulations for preventing and/or treating inflammation and oxidative stress, including the BPH, prostatitis and osteoarthritis.

### Technical state of art. Characteristics of similar technical solutions.

Inflammation, the vascular and cellular response of the tissues against infection, irritation or foreign substances, is one of the most important defense mechanisms of organism, but when this response increases too much may be prejudicial or even fatal. Inflammation represents an important etiological factor in the development of chronic diseases as osteoarthritis, BPH, prostatitis, inflammatory bowel diseases and cancer, among others. The frequency of chronic inflammatory diseases increases with both age and life expectancy.

During inflammation active cells are able to using cell membranes' lipids for generating lipid mediators that act as intracellular or extracellular signals.

Arachidonic acid (AA), a polyunsaturated fatty acid of 20 carbon atoms (5,8,11,14 eicosatetraenoic acid) obtained from foods or by conversion of linoleic acid, is released from membrane phospholipids by the activation of phospholypase A2 enzyme by physical, chemical and biological stimuli (Aster, 2009). The AA derived metabolites named eicosanoids are synthesized by cyclooxygenase (COX) (COX-1 and COX-2) enzymes, which leads to the generation of prostaglandins and thromboxanes, and by lipoxygenase (LOX) enzymes, family of cytosolic enzymes 5-LOX, 15-LOX and 12-LOX, which generate leukotrienes and lipoxins (Diaz, 2001; Gajraj, 2003). Eicosanoids can be involved in different phases of inflammation, so that they are present in the inflammatory exudates and their synthesis is increased in the inflammation sites.

Inflammation is classified in acute or chronic according to the intensity and duration of the response. The acute inflammation, characterized by a short duration, involves three main mechanisms: vasodilatation, increase of capillary permeability and leukocytes migration (mainly neutrophils) from the blood to the inflammatory site, although other events such as phagocytosis, regeneration and cellular repair are also involved (Gallin et al. 1992). The characteristic sings of acute inflammation are redness, swelling, heat, pain and functional impairment (Parakrama and Clive, 2005; Porth, 2007).

On its side, chronic inflammation may last weeks or months and is characterized predominantly by lymphocytes and macrophages infiltration into the lesion site, but eosinophils, mastocytes and neutrophils also are involved. The proliferation of blood vessels and the presence of tissue fibrosis and necrosis are also characteristic of this phase.

Inflammation and oxidative stress are closely related since enzymatic lipid peroxidation (LP) of polyunsaturated fatty acids (PUFAs) produces lipid radical species in a fashion controlled by enzymes implicated in cellular metabolic pathways. Both LOX and COX enzymatic actions are involved within this type of LP, since they catalyse the reactions between 02 and some PUFAs producing lipid hydroperoxides and endoperoxydes. Particularly, LOX enzyme acts catalytically on AA generating lipids hydroperoxides (HPETE) that may be transformed in hydroxylated products (HETE).

On the other hand, although the organic hydroperoxydes thus generated are stable structures they can be broken in front of transition metals (copper and ferrous ions) and generate complex mixtures of secondary peroxidation products like gaseous hydrocarbures (ethane and pentane) and aldehydes (malondialdehyde and 4-hydroxynonenal) that subsequently can damage the cell structure (Halliwell, 1995).

In such regard, a wide variety of synthetic and natural anti-inflammatory agents has been developed in order to ameliorate the inflammation process. Both non-steroidal (NSAID) and steroidal (SAID) anti-inflammatory drugs, therapeutic classes widely prescribed, are well known anti-inflammatory agents very effective for treating acute and chronic inflammation and associated pain.

Nevertheless, these agents produce diverse adverse experiences (AE), among which gastrointestinal, renal and cardiovascular AE are those occurring most commonly in accordance to the specific anti-inflammatory drug (Salles, 2007).

The anti-inflammatory mechanism of NSAID depends on the inhibition of the COX enzymatic pathway, NSAIDs being classified as non-selective or unspecific (which inhibit 1 and 2 COX isoforms) and selective COX-2 inhibitors (Sciulli et al. 2005). The non-selective NSAID (acetyl salicylic acid, indomethacin, naproxen, ibuprophen, diclofenac, among others) effectively ameliorate pain and progression of acute and chronic inflammatory diseases, but their main limitation is the occurrence of gastrointestinal AE, which may be severe when they are administered for long term or to elderly patients. Hypertension and nephrotoxicity also constitute another NSAID-related AE (García and Hernández-Díaz, 2004; Lanas and Fernández, 2006).

Specific COX-2 inhibitors (celecoxib, etoricoxib), drugs of more recent generation, exhibit a high selectivity on COX-2 isoform that supports a better gastric tolerance (Silverstein et al. 2000). However, their use has been associated to severe cardiovascular AE that leaded to withdraw rofecoxib and valdecoxib from the market (Blandizzi et al. 2008).

SAID (prednisone, prednisolone, dexamethasone, among others) produce several AE like hyperglycemia, Cushing syndrome, hypertension, facial redness, vertigo, as well as gastrointestinal and bone (osteoporosis and osteonecrosis that lead to suffer fractures) AE (Mazziotti et al. 2006; Kerachian et al. 2009).

Finally, dual anti-inflammatory drugs (inhibitors of 5-LOX and COX) reduce the progression of chronic inflammatory process without exhibiting the characteristic gastrotoxicity of unspecific NSAID (Whitehouse y Butters, 2003; Whitehouse, 2004).

Benign prostatic hyperplasia (BPH) is a disease whose pathogenesis involves chronic inflammation since its development implicates cellular inflammatory infiltration in the prostate (Roehrborn, 2008; Zhu y McGinley, 2009), so that anti-inflammatory substances could be useful in the treatment of this disease.

BPH, together with cancer and prostatitis, is the most relevant pathology that affects the prostate and deteriorates the quality of life of male adult population, mainly in those in men older than 50 years (Fernández and Pereira, 2008; Alcaraz et al., 2008). BPH consists in the non-malignant and uncontrolled growth of the prostate, a gland that surrounds bladder neck and the first part of the urethra), which when increases in size leads to urethral obstruction. In consequence, most patients with BPH suffer of lower urinary tract symptoms (LUTS) like urinary retention, reduced urinary volume, poor stream, increased micturition latency, irritation, overflow urinary incontinence and nocturia (Nix y Carson, 2007; Roehrborn, 2008; Zhu y McGinley, 2009).

Although the etiology of BPH is multifactorial and not fully understood, it is well documented that it involves both hormonal and non hormonal factors. The main hormonal factor consists in the increased conversion of testosterone in dihydrotestosterone catalyzed by prostate 5 α-reductase, which leads to cell proliferation and prostate growth (static component of BPH), while the main non-hormonal factor consists in the adrenoreceptors (α1-ADR)-mediated increased tone of both the prostate and the bladder smooth muscle (dynamic component of BPH) (Nix and Carson, 2007; Zhuy and McGinley, 2009). While the hormonal component plays a pivotal role in the development of the hyperplasia and prostate enlargement, the increase of the urogenital smooth muscle tone is a key factor in the occurrence of the LUTS (Roehrborn, 2008).

The treatment of BPH includes from watching surveillance to surgery in accordance to the severity of the disease, but pharmacological therapy is that more commonly used, mainly consisting in the use of 5α-reductase inhibitors (finasteride, dutasteride) and α1-ADR (alfuzosin, doxazocin, prazocin, terazocin, tamsulosin) (Doggrell, 2004; Roehrborn et al., 2008; Schwinn and Roehrborn, 2008).

Nevertheless, although the treatment with the former (finasteride, dutasteride) prevents BPH progression and reduce moderately the increase of prostatic volume, not always produces symptom relief and may produce AE, like decreased libido, impotence and ejaculation disorders (Sandhu and Te, 2004; González and Vegas, 2007; Roehrborn, 2008; Roehrborn et al., 2008). On its side, α1-ADR blockers (alfuzosin, doxazosin, prazosin, terazosin, tamsulosin) often alleviate α1-ADR the symptoms without modifying the prostate size, and may produce AE like orthostatic hypotension, fatigue, dizziness, ejaculatory dysfunction and iris muscle alterations (Lepor, 2006; Van Dijk et al., 2006; Bar-Yosef et al., 2008; Prata et al., 2009).

Besides, chronic inflammation and oxidative stress are closely associated with BPH pathogenesis, since increasing evidences demonstrate the relevance of chronic inflammation in the etiology and progression of BPH, so that the use of anti-inflammatory substances has been added to manage this pathology. On the other hand, several evidences suggest a causal relationship between oxidative stress in the prostate tissue and BPH development, and that substances able to produce both a reduction of prostate hyperplasia (PH) and antioxidant effects may provide additional benefits on the hypertrophied prostate (Aydin *et al.,* 2006; Aryal *et al.,* 2007; Siddiqui *et al.,* 2006; Belostottskaia *et al.,* 2006; Prasad *et al.,* 2006).

Phytotherapy with extracts from *Serenoa repens* fruits, *Cucurbita pepo* seeds, the resin of *Prunus africana* and the roots of *Urtica dioca* (Wilt et al., 2000; Koch, 2001; Curtis, 2008) are among the pharmacological treatments to treat the BPH/LUTS entity with a low profile of AE. From them, the lipid extract of *Serenoa repens* (LESR) fruits, which consists in a mixture of free and esterified fatty acids, sterols and fatty alcohols, is that most widely used.

The mechanism of action of LESR is multifactorial and involves several of the factors referred above, like the inhibition of 5α-reductase (Raynaud et al., 2000; Abe et al., 2009), α1-ADR antagonism (Gerber et al., 2001; Debruyne et al., 2002; Abe et al., 2009), its anti-inflammatory effect mediated by the inhibition of COX and 5-LOX (Mogul et al., 2000; Potenziani, 2003; Raman et al., 2007; Curtis, 2008) and its antioxidant effects (Henry, 2000; Perona, 2005; Chapkin et al., 2007), among others. Although preclinical toxicology data on LESR are scarce, it is well tolerated and shows a low frequency of AE (Rockville, 2005).

D-004 is another lipid extract with potential value for treating BPH that has been recently developed from *Roystonea regia* fruits. The mechanism of action that supports its efficacy on experimental models of PH is multifactorial and involves the competitive inhibition of prostate 5α-reductase and the non-competitive antagonism of the α1-ADR-mediated contractile response of the prostate tissue. Additionally, D-004 produces pleiotropics anti-inflammatory (linked to both COX and 5-LOX inhibition) and antioxidant effects (on both normal and hypertrophied prostate tissues), which potentially could contribute to its efficacy (Menéndez, 2006; Pérez, 2008).

Osteoarthritis (OA), the most common of degenerative arthritis, is another pathology that involves inflammatory processes and that is characterized by the unbalance between the synthesis and degradation of the cartilage matrix (made of proteoglycans, collagen and water), cartilage destruction, impairment of subchondral bone and further inflammatory response, wherein the cartilage not only does not regenerate, but may disappear (Little, 2008).

In the past, research on OA was focused on cartilage degeneration since it was considered as the most relevant underlying disease change, but currently it is accepted that beyond the cartilage, OA involves all joint components, like the subchondral bone, ligaments, joint capsule, synovial membrane, periarticular muscle, meniscus and nervous terminations (Lotz, 2010; Heinegard, 2011). The response of subchondral bone in OA includes the production of new bone that leads to develop marginal osteophytes that protrude as nodules that may be secondarily inflamed or as bony outgrowths able to irritate neighbor structures (Buckland-Wright, 2004; Little, 2008).

The main drugs used for treating OA are those that alleviate the pain and improve the functional incapacity, like NSAID and SAID, are those more used for treating OA, but they do not modify the course of the disease since they do not prevent the damage of cartilage (McMahon, 2008). Antiresortive drugs have been also used to manage OA, since alendronate and estrogens have demonstrated to significantly reduce subchondral bone lesions in older women with knee OA as compared to non-treated older women (Carbone, 2004), which supports that OA involves all joint components (cartilage and bone).

The increase of oxidative stress, the unbalance between the cell metabolism-induced production of reactive oxygen species (ROS) and free radicals (FR), and the ability of the antioxidant systems for counteracting such production and/or effects, is seen as a contributing factor to OA pathogenesis, which involves an increase of LP and a decrease of endogen antioxidant defenses like vitamin C and superoxide dismutase activity (SOD) (Manesh, 2005; Chandran, 2009).

Nevertheless, the obtainment of active ingredients from the fruits of *Acrocomia crispa* (A, crispa) and/or *Acrocomia aculeata* (*A. aculeata*) species with antioxidant effects and effective to prevent and treat inflammatory processes, included those previously referred, had not been reported before; nor also a simple and economic method for obtaining this type of substance.

The active ingredient claimed in this invention surpasses in efficacy and potency to the extracts of natural origin above mentioned, without showing the AE of synthetic drugs.

### Divulgation of the Invention

The procedure for obtaining the active ingredient, object of the current invention, employ green or ripe fruits of *A. crispa* and/or *A. Aculeata* species, both of the Arecaceae family, which are dried well at ambient conditions or in ovens, and grounded in a hammer, blade or laminator disc mill, up to obtain a particle size < 3 mm. A lipid active ingredient is obtained from such dry and ground material, which is mainly composed of free fatty acids, but that also may contain sterols and fatty alcohols in lower proportions. The obtainment of this active ingredient may include an enzymatic, basic or acid hydrolysis (total or partial) with a further recovery of the fatty acids; or well a selective extraction with organic solvents (hydrocarbons, alcohols, ethyl acetate, acetone or mixtures of them) or an extraction with supercritical fluid. The hydrolysis process can be applied directly to the ground vegetable material or to the lipid active ingredient obtained from it.

For obtaining the active ingredient with most fatty acids as acylglycerols or ethyl esters, the dry and ground vegetal material is submitted to selective extraction with organic solvents, followed by the further filtration and elimination of the solvent with heating at low pressure or to an extraction in supercritical conditions. The extraction process may be accomplished in a conventional solid-liquid extractor like a shaking reactor or a soxhlet apparatus, where the fatty acids (present as acylglycerols, ethyl esters and in less proportions free) are separated from other components present in the vegetable material through a selective extraction in the adequate solvent, like alcohols between 1 and 3 carbon atoms (methanol, ethanol, 2-propanol), hydrocarbons between 5 to 8 carbon atoms (pentane, isopentane, hexane, heptane and octane), ethyl acetate, acetone, or mixtures of them; although can be also accomplished by a supercritical extraction with CO₂. The ethanol extraction favors the obtainment of ethyl esters, which are fairly present in little proportions in the fruits, but favored in an acidic media.

For obtaining the active ingredient with the fatty acids in free form, a hydrolysis process is required, which may be enzymatic (with lipases), basic (with alkaline, alkaline-earthen, organic hydroxides or ammonium hydroxide) or acid (with hydrochloric, citric or sulfuric acid). The hydrolysis, which could be total or partial, may be done to the ground vegetable material or to the oily extract obtained from it, and the free fatty acids are recovered thereafter. When basic hydrolysis is used, proper agitation in a diluted acid media (by using hydrochloric, citric or sulfuric acids) makes possible the release of fatty acids, which separated from the aqueous phase by forming an upper oily phase, which is in turn washed with treated water and dried with heat at low pressure.

By this method, a 10 to 40 % yield from the dry and ground vegetable material is achieved, ensuring the obtainment of this active ingredient, which is mainly composed by free or esterified (like acylglycerols or ethyl esters) fatty acids, mainly saturated acids of 8, 10, 12, 14, 16 and 18 carbon atoms, and unsaturated acids of 16 and 18 carbon atoms, but that can also contain sterols and fatty alcohols. The proportion of fatty acids in the mixture (free or esterified) is shown in Table 1.

**Table 1. Content of fatty acids in the active ingredient**

| **Component** | **Relative Content (%)** |
|---|---|
| caprilic acid (C_{8:0}) | < 3.0 |
| capric acid (C_{10:0}) | < 3.0 |
| lauric acid (C_{12:0}) | 30.0 - 40.0 |
| myristic acid (C_{14:0}) | 8.0 - 15.0 |
| palmitic acid (C_{16:0}) | 5.0 - 12.0 |
| palmitoleic acid (C_{16:1}) | < 3.0 |
| stearic acid (C_{18:0}) | 1.0 - 5.0 |
| oleic acid (C_{18:1})* | 20.0 - 35.0* |

| | |
|---|---|
| * Determined and reported as oleic acid, includes the mixture of oleic C_{18:1}(60 - 70 %); linoleic C_{18:2}(25 - 40 %) and linolenic C_{18:3} (< 10 %) acids. | |

An overall assessment let to affirm that this substance or active ingredient is more effective and potent than other currently known active ingredients from vegetable origin for prevention and/or treatment of inflammatory process and oxidative stress, including BPH, prostatitis and osteoarthritis. This active ingredient has shown absence of toxicity in rodent studies, and has been well tolerated in humans, which represents an advantage for its potential use.

### Examples of execution.

The objectives of the present invention should be described in detail below, by using examples that do not limit its scope.

### Example 1

Fresh fruits (5 kg) of *A. crispa* were taken and placed in an oven at controlled temperature, 60 °C for 7 days and further milled in a blade mill up to obtain a particle size between 1500 and 2000 µm. Then 1000 g of this powder were taken, placed in a shaking reactor and extracted with 10 L hexane at 55 °C by using constant shaking for 16 hours, this process being repeated three times. Then, the product was filtered and evaporated to dryness at 50 °C in a vacuum-assisted evaporator. The obtained substance, which weighed 120 g, had the composition shown in table 2, determined by using gas chromatography. When the same procedure was applied to *A. aculeata* fruits, 150 g of active ingredient were obtained.

**Table 2. Composition of fatty acids (%) in the active ingredient**

| **Component** | **Active Ingredient obtained from *A*. *crispa* (%)** | **Active Ingredient obtained from *A*. *aculeata* (%)** |
|---|---|---|
| caprilic acid (C_{8:0}) | 1.6 | 1.3 |
| capric acid (C_{10:0}) | 0.9 | 0.7 |
| lauric acid (C_{12:0}) | 32.2 | 31.8 |
| myristic acid (C_{14:0}) | 14.7 | 14.5 |
| palmitic acid (C_{16:0}) | 11.5 | 11.7 |
| palmitoleic acid (C_{16:1}) | 0.5 | 0.8 |
| stearic acid (C_{18:0}) | 4.4 | 4.6 |
| oleic acid (C_{18:1})* | 34.2 | 34.6 |

| | | |
|---|---|---|
| * Determined and reported as oleic acid, includes the mixture of oleic C_{18:1}; linoleic C_{18:2} and linolenic C_{18:3} acids. | | |

### Example 2

Fresh fruits (10 kg) of *A. aculeata* were taken, dried at ambient conditions for 15 days, and then ground to a particle size < 1500 µm by using a hammer grinding mill. One thousand (1000) g of this powder were taken and submitted to alkaline hydrolysis. Fatty acids were released with 30% HCl, the organic phase was extracted and washed 5 times with water, filtered and evaporated to dryness at 80 °C in a vacuum-assisted evaporator. The obtained substance was weighed (200 g) and then analysed by gas chromatography, resulting the composition shown in table 3. When the same procedure was applied to *A*. *crispa* fruits, 230 g of active ingredient were obtained.

**Table 3. Composition of fatty acids (%) in the active ingredient**

| **Component** | **Active Ingredient obtained from *A*. *crispa* (%)** | **Active Ingredient obtained from *A*. *aculeata* (%)** |
|---|---|---|
| caprilic acid (C_{8:0}) | 1.2 | 0.9 |
| capric acid (C_{10:0}) | 1.9 | 1.7 |
| lauric acid (C_{12:0}) | 37.8 | 39.0 |
| myristic acid (C_{14:0}) | 14.3 | 14.9 |
| palmitic acid (C_{16:0}) | 10.9 | 11.3 |
| palmitoleic acid (C_{16:1}) | 0.2 | 1.4 |
| stearic acid (C_{18:0}) | 3.3 | 3.6 |
| oleic acid (C_{18:1})* | 30.4 | 27.2 |

| | | |
|---|---|---|
| * Determined and reported as oleic acid, includes the mixture of oleic C_{18:1}; linoleic C_{18:2} and linolenic C_{18:3} acids. | | |

### Example 3

Fresh fruits (5 kg) of *A. crispa* and (5 kg) of *A. aculeata* were taken, dried in an oven at controlled temperature (45 °C) for 7 days, and then ground in a grinding mill up to reach a particle size between 1500 and 1800 µm. One thousand (1000) g of this powder were taken and submitted to continuous supercritical fluid extraction with 10 L CO₂ at 35 °C and 250 bars, for 16 hours. Then, the product was submitted to enzymatic hydrolysis, weighed (150 g) and analysed by gas chromatography, which shows the composition summarized in table 4.

**Table 4. Composition of fatty acids (%) in the active ingredient**

| **Component** | **Active Ingredient obtained from *A*. *crispa* (%)** | **Active Ingredient obtained from *A*. *aculeata* (%)** |
|---|---|---|
| caprílic acid (C8:0) | 2.0 | 1.5 |
| capric acid (C10:0) | 1.5 | 2.2 |
| lauric acid (C12:0) | 32.5 | 32.7 |
| myristic acid (C14:0) | 13.8 | 14.1 |
| palmitic acid (C16:0) | 11.9 | 11.6 |
| palmitoleic acid (C16:1) | 1.3 | 1.8 |
| estearic acid (C18:0) | 2.6 | 1.9 |
| oleic acid (C18:1)* | 34.4 | 34.2 |

| | | |
|---|---|---|
| * Determined and reported as oleic acid, includes the mixture of oleic C18:1; linoleic C18:2 and linolenic C18:3 acids. | | |

### Example 4

Fresh fruits (10 kg) of *A. crispa* were taken, dried at ambient conditions for 20 days, and milled in a laminator disc mill up to a particle size < 1000 µm. One thousand (1000) g of this powder were taken, placed in a soxhlet apparatus, extracted with 10 L ethanol for 36 hours and then filtered and evaporated to dryness at 80 °C by using a vacuum-assisted evaporator. After solvent remotion, a partial hydrolysis with an ammonium hydroxide solution at 60 °C was carried out. The fatty acids were then released with 10% H₂SO₄ and this extract was washed with water, dried at vacuum, weighed (300 g) and analysed by gas chromatography, its composition being shown in table 5. When the same procedure was applied to *A. aculeata* fruits, 280 g of the active ingredient were obtained.

**Table 5. Composition of fatty acids (%) in the active ingredient**

| **Component** | **Active Ingredient obtained from *A*. *crispa* (%)** | **Active Ingredient obtained from *A*. *aculeata* (%)** |
|---|---|---|
| caprilic acid (C_{8:0}) | 0.9 | 0.7 |
| capric acid (C_{10:0}) | 1.5 | 1.8 |
| lauric acid (C_{12:0}) | 39.8 | 39.9 |
| myristic acid (C_{14:0}) | 14.3 | 14.1 |
| palmitic acid (C_{16:0}) | 7.4 | 8.6 |
| palmitoleic acid (C_{16:1}) | 1.2 | 0.9 |
| stearic acid (C_{18:0}) | 1.1 | 1.3 |
| oleic acid (C_{18:1})* | 33.8 | 31.7 |

| | | |
|---|---|---|
| * Determined and reported as oleic acid, includes the mixture of oleic C_{18:1}; linoleic C_{18:2} and linolenic C_{18:3} acids. | | |

### Example 5

Fresh fruits (10 kg) of *A. crispa* were taken, dried at ambient conditions for 20 days, and then milled in a laminator disc mill up to a particle size < 1000 µm. One thousand (1000) g were taken, extracted with 10 L ethanol in an agitated reactor at 65 °C for 36 hours, filtered, evaporated to dryness at 80 °C at reduced pressure and then hydrolysed with HCl after solvent remotion. The obtained extract was washed with water, dried at vacuum, weighed (250 g) and analysed by gas chromatography, its composition being shown in table 6. When the same procedure was applied to *A. aculeata* fruits, 220 g of active ingredient were obtained.

**Table 6. Composition of fatty acids (%) in the active ingredient**

| **Component** | **Active Ingredient obtained from *A*. *crispa* (%)** | **Active Ingredient obtained from *A*. *aculeata* (%)** |
|---|---|---|
| caprilic acid (C_{8:0}) | 1.1 | 0.8 |
| capric acid (C_{10:0}) | 1.3 | 1.3 |
| lauric acid (C_{12:0}) | 39.0 | 39.3 |
| myristic acid (C_{14:0}) | 14.6 | 14.0 |
| palmitic acid (C_{16:0}) | 7.1 | 8.1 |
| palmitoleic acid (C_{16:1}) | 1.1 | 1.8 |
| stearic acid (C_{18:0}) | 1.2 | 1.1 |
| oleic acid (C_{18:1})* | 34.6 | 33.6 |

| | | |
|---|---|---|
| * Determined and reported as oleic acid, includes the mixture of oleic C_{18:1}; linoleic C_{18:2} and linolenic C_{18:3} acids. | | |

### Example 6

The effect of the active ingredients referred in example 1 was evaluated in a chronic inflammation model (cotton granuloma) for which male Sprague Dawley rats (250 - 270 g) were adapted for 7 days to laboratory conditions. Once concluded the adaptation period rats were randomized into different groups, anesthetized with sodium thiopental (30 mg/kg, i.p) and their dorsal region disinfected with 70% alcohol solution; an incision in the middle dorsum lateral region was done with blunt nippers thus creating a subcutaneous tunnel wherein a sterile cotton pellet of 50 mg was implanted. The wound was sutured and then an antiseptic solution was applied locally. After 18 hours of the last administration the rats were sacrificed in ether atmosphere, the granulomas were carefully dissected, removed and dried at 60 °C for 24 hours up to achieve constant weight. Dry granuloma weight was calculated by the difference between the weight of the dry granuloma and the weight of the implanted cotton pellets (50 mg). The inhibition degree (%) was estimated assuming that the granuloma weight in the positive control group was 100%.

The extracts of A. crispa and A. aculeata fruits were suspended in Tween 20/water vehicle. Rats were randomly distributed into seven groups (10 rats/group): a vehicle control group, 3 groups treated with *A. crispa* and 3 with *A. aculeata* extracts (25, 200 y 500 mg/kg in both cases, respectively). The administration was done by gastric gavage (5 mL/kg) for the next 6 days following cotton pellet implantation and then the granuloma dry weigh was quantified. Comparisons between control and treated groups were performed by Mann Whitney U test.

The subcutaneous implantation of cotton pellet in the middle lateral dorsal region of the animals induced the granuloma formation (Table 7). All treatments significantly reduced the granuloma dry weight as compared to that of the control group. The effects of *A. crispa* and *A. aculeata* fruits extracts increased with the doses to 54.6 and 55.4 % of inhibition, respectively, with the dose of 500 mg/kg.
The results of the present work demonstrate the efficacy of *A. crispa* and *A. aculeate* fruit extracts in the model of cotton pellet granuloma in rats, a significant reduction of the granuloma weight being observed, which shows its potential therapeutic as anti-inflammatory.

**Table 7. Effects of A. crispa and A. aculeata extracts in the model of cotton pellet granuloma in rats.**

| Treatments | Doses (mg/kg) | Granuloma dry weight (mg) | I (%) |
|---|---|---|---|
| Control (vehicle + granuloma) | 0 | 258.0 ± 0.12 | - |
| | | | |
| *Acrocomia crispa* + granuloma | 25 | 138.0 ± 0.05* | 46.5 |
| *Acrocomia crispa* + granuloma | 200 | 132.0 ± 0.11* | 48.8 |
| *Acrocomia crispa* + granuloma | 500 | 117.0 ± 0.04 ** | 54.6 |
| | | | |
| *Acrocomia aculeata* + granuloma | 25 | 135.0 ± 0.05* | 47.6 |
| *Acrocomia aculeata* + granuloma | 200 | 130.0 ± 0.11* | 49.6 |
| *Acrocomia aculeata* + granuloma | 500 | 115.0 ± 0.04 ** | 55.4 |

| | | | |
|---|---|---|---|
| Values expressed as mean ± SME (standard mean error); I (%): percentage of inhibition * p < 0,05; ** p < 0,01 Comparison with Control, (Mann Whitney U test) | | | |

### Example 7

The effect of the active ingredient mentioned in the example 2 was assessed in a model of acute inflammation (carragenan-induced pleurisy). Male Sprague Dawley rats (190-290 g) were adapted to laboratory conditions during seven days and after that were randomly distributed in different groups.

Carrageen-induced pleurisy was provoked 1 hour after the oral administration of the treatments. In brief, rats were anesthetized in ether atmosphere. Pleurisy was induced in rats by intrapleural administration of 300 µL of 1% (w/v) carrageen suspension in saline solution. A specially adapted needle was introduced into the right side of the thoracic cavity of rats to inject the carrageen suspension, and an equal volume of sterile saline was injected into the negative controls. Five hours later, under ether anesthesia, rats were sacrificed, their thoracic cavities were opened and pleural exudates were collected, homogenized in 1 mL of 3.15% sodium citrate and measured in graduated plastic tubes as exudates volume (EV). Blood- contaminated exudates was discarded.

The fruit extracts of *A. crispa* and *A. aculeata* were suspended in Tween-20/water solution, being administered as single doses by gastric gavage (5 ml/kg). The rats were randomly distributed in eight groups (10 rats/group): a negative control group that was injected with saline solution in pleural cavity and seven groups that were injected with carrageen: a positive control group treated with vehicle, three groups treated with extracts of *A. crispa* and three with *A. aculeata* (25, 200 and 500 mg/kg), respectively. The comparison between control and treated groups was performed by Mann Whitney *U* test.

The carrageen injection produced a significant increase of EV and MPO activity in the exudates of positive control group compared to the negative control group (Table 8).

The administration of single oral doses of both substances (25, 200, 500 mg/kg), objective of the present invention, was effective for significantly and dose dependently reducing EV and inhibiting the enzymatic activity of MPO, achieving an inhibition percentage > 50% respect to control group with the major assayed doses (500 mg/kg).

The results demonstrated the efficacy of fruit extracts of *A. crispa* and *A. aculeata* in the carrageen induced pleurisy in rats, with a significant and dose-dependently reduction of EV and MPO activity, indicating its potential anti-inflammatory effect.

**Table 8. Effect of oral treatment with A. crispa and A. aculeata extracts on EV and MPO in pleural exudates of rats with carrageen induced pleurisy.**

| Treatment | Doses (mg/kg) | EV (mL) | I (%) | MPO (U/mg of protein) | I (%) |
|---|---|---|---|---|---|
| Negative control (vehicle) | 0 | 0.01 ± 0.01*** | - | 0.37 ± 0.16*** | - |
| Positive control (vehicle + C) | 0 | 1.45 ± 0.03 | - | 43.58 ± 1.84 | - |
| *Acrocomia crispa* + *C* | 25 | 1.12 ± 0.06** | 22.9 | 36.70 ± 1.25* | 15.9 |
| *Acrocomia crispa* + C | 200 | 0.95 ± 0.06*** | 34.7 | 29.93 ± 0.97*** | 31.6 |
| *Acrocomia crispa* + C | 500 | 0.73 ± 0.05*** | 50.0 | 21.82 ± 1.11*** | 50.0 |
| *Acrocomia aculeata* + C | 25 | 1.11 ± 0.06** | 23.6 | 35.35 ± 1.27* | 19.0 |
| *Acrocomia aculeata* + C | 200 | 0.94 ± 0.05*** | 35.4 | 29.99 ± 0.97*** | 31.4 |
| *Acrocomia aculeata* + C | 500 | 0.73 ± 0.05*** | 50.0 | 21.85 ± 1.15*** | 50.0 |

| | | | | | |
|---|---|---|---|---|---|
| Values expressed as Mean ± SME (standard mean error); EV: exudates volume; MPO: mieloperoxidase; I (%): percentage of inhibition; C: carrageen. *p<0,05; **p<0,01; ***p<0,001. Comparison with positive control group (Mann Whitney U test) | | | | | |

### Example 8

Comparative study of the effects of active ingredients referred in the example 3 with the lipid extracts of fruits of *S. repens* (LESR) and *R. regia* (D-004) in a model of acute inflammation (edema induced by xylene in ear of mice).

In order to perform this study the male young mice OF1 (20-30 g) were adapted during 7 days at laboratory conditions. After adaptation period, the mice were randomized distributed in six groups: a negative control group (vehicle), a positive control group (vehicle + xylene application), and four groups treated with doses of 400 mg/kg of extract of *A. crispa, A. aculeate,* LESR and D-004 that were applied with xylene. All treatments were administered 1 hour before edema induction.

The edema induction was performed by topical application of 30 µL of xylene in the dorsal face of right ear of mice. Two hours later, edema was quantified, for that the mice were anesthetized in ether atmosphere and were sacrificed by cervical dislocation. Both ear were cut and weighed in an Analytical Balance (Mettler Toledo). The formation of edema was calculated by difference between the weights (mg) of right (with edema) and left ear (without edema) (ΔO). The comparison between control and treated groups was performed by the Mann Whitney *U* test.

The topical application of xylene significantly increased the difference of weight between both ears, indicating an edema formation compared to negative control group (Table 9). All treatments significantly inhibited the increase of edema. The extracts of *A. crispa* and *A. aculeata* were more effective (54.5 and 52.9 % of reduction, respectively) than LESR (26.7 % of reduction) and D-004 (34 % of reduction).

These results indicate that the efficacy of oral treatment with fruit extracts of *A. crispa* and *A. aculeata* (400 mg/kg) on edema induction by xylene topical application in ear of mice was superior to similar doses of LESR and D-004.

**Table 9. Effect of Acrocomia crispa, Acrocomia aculeata extracts, LESR and D004 on edema induced by xylene in ear of mice.**

| Treatment | Doses (mg/kg) | Edema (ΔO) | I (%) |
|---|---|---|---|
| Negative control | 0 | 2.67 ± 1.08*** | - |
| Positive Control + xylene | 0 | 37.87 ± 5.34 | - |
| *Acrocomia crispa* + xylene | 400 | 18.67 ± 3.25** | 54.5 |
| *Acrocomia aculeata* + xylene | 400 | 19.25 ± 3.71** | 52.9 |
| LESR + xylene | 400 | 28.48 ± 2.74*+† | 26.7 |
| D004+ xylene | 400 | 25.89 ± 2.36*+† | 34.0 |

| | | | |
|---|---|---|---|
| Values expressed as Mean ± SME (standard mean error); I (%): percentage of inhibition. * p< 0.05, **p<0.01 comparison with positive control, + p< 0.05 comparison vs *Acrocomia crispa;* † p< 0.05 comparison vs *Acrocomia aculeata* (Mann Whitney *U* test) | | | |

### Example 9

In order to evaluate the antioxidant effect of active ingredient referred in the example 4 male Sprague Dawley rats (200-250 g of body weight) were adapted to laboratory conditions during 7 days. After adaptation period the animals were randomly distributed in different groups, anesthetized in ether atmosphere and bleeding by abdominal aorta.

The blood was collected with EDTA (10%) (final concentration: 1 mg/ml of blood). The plasma was obtained by centrifugation at 3000 rpm during 10 min and was used for determining the malondialdehyde (MDA) and sulphydril groups (SHG) concentrations (LP and protein oxidation markers, respectively).

The *A. crispa* and *A. aculeata* fruit extracts were suspended in solution of Tween-20/water. The rats were randomly distributed in 9 groups (10 rats/group): a control treated with vehicle, 4 with extracts of *A. crispa* and 4 with extracts of *A. aculeata* (5, 25, 50 and 200 mg/kg). The treatments were administered by gastric gavage during 30 days. After that, MDA and SHG plasma concentrations were biochemically determined. The comparisons between control and treated groups were performed using Mann Whitney *U* test.

As observed in the table 10, both treatments significantly and markedly reduced the plasma values of MDA and SHG. The treatment with *A. crispa* achieved reductions of 63.4% (MDA) and 59.4% (SHG). The results of the present study demonstrate the efficacy of fruit extracts of *A. crispa* and *A. aculeata* for reducing the MDA and SHG plasma levels, demonstrating the antioxidant effects of these extracts.

**Table 10. Effect of oral treatment with A. crispa and A. aculeata extracts on plasma values of MDA and SHG in rats.**

| Treatment | Doses (mg/kg) | MDA (nmol of MDA/mg of protein) | I (%) | SHG (mmol) | I (%) |
|---|---|---|---|---|---|
| Control | | 100.75 ± 3.13 | - | 1.48 ± 0.10 | - |
| *Acrocomia crispa* | 5 | 81.57 ± 3.48* | 19.0 | 1.27 ± 0.14 | 14.1 |
| *Acrocomia crispa* | 25 | 71.33 ± 3.51* | 29.2 | 0.88 ± 0.04* | 40.5 |
| *Acrocomia crispa* | 50 | 38.57 ± 7.66** | 61.7 | 0.72 ± 0.04** | 51.3 |
| *Acrocomia crispa* | 200 | 36.01 ± 5.22** | 64.2 | 0.61 ± 0.05** | 58.7 |
| | | | | | |
| *Acrocomia aculeata* | 5 | 82.57 ± 3.48* | 18.0 | 1.25 ± 0.11 | 15.5 |
| *Acrocomia aculeata* | 25 | 70.14 ± 3.08* | 30.3 | 0.86 ± 0.03* | 41.8 |
| *Acrocomia aculeata* | 50 | 37.71 ± 7.31** | 62.5 | 0.70 ± 0.07** | 52.7 |
| *Acrocomia aculeata* | 200 | 36.8 ± 5.33** | 63.4 | 0.60 ± 0.06** | 59.4 |

| | | | | | |
|---|---|---|---|---|---|
| Values expressed as Mean ± SME (standard mean error); I (%): percentage of inhibition. * p< 0.05, **p<0.01 comparison with control group (Mann Whitney U test) | | | | | |

### Example 10

In order to evaluate the possible effect of active ingredient referred in the example 5 on prostate hyperplasia in rats, active ingredients were suspended in vehicle Tween 20/water. Male Sprague Dawley rats (300 - 360 g) adapted during 7 days to laboratory conditions were randomly distributed in eight experimental groups: a negative control group (vehicle) and 7 groups with prostate hyperplasia induced by testosterone injection (4 mg/kg): a positive control that only received vehicle and 6 groups treated with extracts of *A. crispa* and *A. aculeata* (50, 200 y 400 mg/kg). The testosterone propionate was dissolved in vegetable oil and subcutaneously injected, daily, during 14 days.

The oral treatments were administered by gastric gavage (5 ml/kg) once a day during 14 days. The rats were sacrificed under ether atmosphere, bleeding and opened its abdomen by incision in the ventral middle lineal; prostates and bladder were dissected, removed and weighed. The comparison between control and treated groups were performed using Mann Whitney U test.

The testosterone injection produced a significant increase of prostate weight and prostate weight/ body weight ratio compared to negative control group (Table 11). The oral administration of fruit extracts of *A. crispa* and *A. aculeata* during 14 days to rats significantly reduced the increase of prostate size induced by testosterone injection (≅ 44 % with the major doses assayed).

These results indicate the efficacy of the fruit extracts of *A. crispa* and *A. aculeata* for significantly and markedly reducing the increase of prostate size induced by testosterone in rodents, which indicate the potential effect of these extracts on prostate hyperplasia.

**Table 11. Effect of A. crispa and A. aculeata extracts on testosterone induced prostate hyperplasia in rats.**

| Treatment | Doses (mg/kg) | BW | PW | I (%) | PW/BW | I (%) |
|---|---|---|---|---|---|---|
| Negative Control | 0 | 359,7 ± 6,9 | 576,03 ± 51,4*** | - | 1,60± 0,15*** | - |
| Positive Control | 0 | 338,5 ± 6,5 | 916,07 ± 34,4 | - | 2,70 ± 0,12 | - |
| | | | | | | |
| *Acrocomia crispa* + T | 50 | 338,5 ±10,2 | 895,4 ± 34,6 | 6,0 | 2,64 ± 0,11 | 5,45 |
| *Acrocomia crispa* + T | 200 | 335,3 ±6,4 | 838,05 ± 30,9* | 23,0 | 2,50 ± 0,09 | 18, 1 |
| *Acrocomia crispa* + T | 400 | 349,0 ± 6,4 | 775,02 ± 31,1 ** | 41,4 | 2,22 ± 0,06 ** | 43,6 |
| | | | | | | |
| *Acrocomia aculeata* + T | 50 | 342,8 ±10,2 | 890,8 ± 35,1 | 7,4 | 2,60 ± 0,12 | 9,0 |
| *Acrocomia aculeata*+ T | 200 | 332,1 ±6,4 | 840,03 ± 31,0* | 22,3 | 2,52 ± 0,15 | 16,3 |
| *Acrocomia aculeata* + T | 400 | 350,4 ± 6,4 | 776,01 ± 35,4 ** | 41,1 | 2,21 ± 0,11 ** | 44,5 |

| | | | | | | |
|---|---|---|---|---|---|---|
| Values expressed as Mean ± SME (standard mean error); T: testosterone; BW: body weight (g); PW: prostate weight (mg); I (%): percentage of inhibition. * p< 0.05, **p<0.01 comparison with positive control (Mann Whitney U test) | | | | | | |

### Example 11

The effect of active ingredient referred in example 1 in a model of osteoarthritis was evaluated in the arthritis model induced by monoiodoacetate (MIA) in rats. Male Sprague Dawley rats (150-175 g), adapted to laboratory conditions during 7 days, were randomly distributed in 8 experimental groups: a negative control (vehicle) and 7 groups with MIA induced osteoarthritis: a positive control that only received vehicle and 6 treated groups with fruit extracts of *A. crispa* and *A. aculeata* (100, 200 y 400 mg/kg).

The arthritic damage was induced by single injection of MIA (50 µl) in the synovial cavity of the left knee. An equivalent volume of saline solution was injected in the right knee. The treatments were administered by gastric gavage (5 mL/kg) immediately after damage was induced. After that, the rats were sacrificed under anesthesia.

The articulation of left knee was extracted and stored in formol during 24 hour with the objective of performing the histological study. Later on the samples were decalcified in EDTA disodium 0.5 M (pH 7.4) at 4°C during 4 weeks. At decalcification completion the articulations were sectioned in the longitudinal flat for obtaining two half, being furthering included in paraffin, cut and stained with hematoxiline, eosine and toluidine blue for analyzing the cartilages. The damage of cartilage was evaluated according to depth and extension using a score modified of Mankin as following:
- **Depth (0-5):** 0-normal, 1- minimum, affecting only the superficial zone, 2-light invasion to mediate superior zone, 3-moderated invasion also in mediate zone, 4-marked invasion to depth zone, but not to divisor line or interface between no-calcified and calcified cartilage (tidemark) and 5-severe degradation of all thickness up to divisor line present between no-calcified and calcified cartilage.
- **Reduction of coloration of matrix (0-4):** 0- normal/light reduction of coloration, 1-reduced coloration in the radial layer, 2- reduced coloration in the inter-territorial matrix, 3- coloration only present in extracellular matrix, and 4- absent coloration.
- **Changes of cartilage (0-6):** 0- normal, 1-irregular surface, including fissures in the radial layer, 2- pannus, 3- absence of superficial layers of cartilage, 4- light disorganization (cellular column absent, some little superficial conglomerates), 5- fissure in the layer of calcified cartilage, and 6- disorganization (chaotic structure, conglomerates and osteoclastic activity).
- **Extension of inflammation (0-4):** 0- normal (non-infiltrated), 1- minim inflammatory infiltration, 2-light infiltration, 3- moderate infiltration and 4-marked infiltration.
- **Cellular abnormalities (0-3):** 0-normal, 1- hypercellularity, including small superficial conglomerates, 2- conglomerates and 3-hypocellularity.
- **Extension of damage to the tibia (0-3):** 0-normal, 1-minim, moderate and 3-severe.
- **Loss of cortical or trabecular bone (0-4):** 0-normal, 1-minimal loss of cortical bone in a few sites, 2-light loss of cortical or trabecular bone, 3-moderate loss of bone in many sites and 4-marked loss of bone in many sites with fragmentation and penetration in all thickness of inflammatory process of pannus in the cortical bone.
- **Presence of osteoclasts (0-4):** 0- normal (essentially no osteoclasts), 1-few osteoclasts (lined in the 5% of the majority of affected surface of bone), 2- some osteoclasts (lined in 5 and 25 % of the majority of affected surface of bone), 3- many osteoclasts (lined between 26-50 % of the mayoralty of affected surface of bone) and 4- great quantity of osteoclasts (lined in more of 50% of the affected surface of bone).

The media of scores of studied histological parameters were calculated. The histological score was calculated as the average of all the histological variables evaluated. The comparisons between control and treated groups were performed with the Mann Whitney U test.
None animal of negative control group and all animals of positive control group showed evidences of damage according to studied variables (Table 12, 13 and 14). The administration of *A. crispa* and *A. aculeata* fruit extracts significantly reduced the increase of histological score induced by MIA in the articulation in the knee (50% with the major assayed doses).
These results indicate the efficacy of *A. crispa* and *A. aculeata* fruit extracts for significantly and markedly reducing the damage in synovial cavity of left knee of rats induced by single injection of MIA, which indicates their potential therapeutic effect on the osteoarthritis.

**Table 12. Effects of A. crispa and A. aculeata extracts on total histological score.**

| Treatments | Doses (mg/kg) | Histological score | I (%) |
|---|---|---|---|
| Negative control | 0 | 0** | - |
| Positive control | 0 | 3.55 ± 0.22 | - |
| | | | |
| *Acrocomia crispa* | 100 | 2.39 ± 0.24** | 32.7 |
| *Acrocomia crispa* | 200 | 1.98 ± 0.27*** | 44.2 |
| *Acrocomia crispa* | 400 | 1.75 ± 0.20*** | 50.1 |
| | | | |
| *Acrocomia aculeata* | 100 | 2.37 ± 0.21*** | 33.2 |
| *Acrocomia aculeata* | 200 | 1.97 ± 0.23*** | 44.5 |
| *Acrocomia aculeata* | 400 | 1.76 ± 0.27*** | 50.0 |

| | | | |
|---|---|---|---|
| Positive control and all treated groups with the extracts received injections of MIA Values expressed as Mean ± SME (standard mean error); I (%): percentage of inhibition. * p< 0.05, **p<0.01 comparison with positive control (Mann Whitney U test) | | | |

**Table 13. Effects of A. crispa and A. aculeata extracts on damage of cartilage**

| Treatments | Depth of damage | | Reduction of coloration of matrix | |
|---|---|---|---|---|
| | | I (%) | | I (%) |
| Negative control | 0** | | 0** | |
| Positive Control | 4.63±0.52 | | 3.00±0.0 | |
| | | | | |
| *A. crispa* 100 mg/kg | 2.63±0.5 | 43.2 | 1.88±0.6** | 37.3 |
| *A. crispa* 200 mg/kg | 2.38±0.5* | 48.6 | | 50.0 |
| *A. crispa* 400 mg/kg | 2.05±0.6 | 55.7 | 1.15±0.5*** | 61.6 |
| | | | | |
| *A. aculeata* 100 mg/kg | 2.61± 0.5 | 43.6 | 1.89±0.6** | 37.0 |
| *A. aculeata* 200 mg/kg | 2.37±0.b | 48.8 | 1.51±0.5*** | 49.6 |
| *A. aculeata* 400 mg/kg | 2.03±0.61 | 56.1 | 1.22±0.6*** | 59.3 |

| | | | | |
|---|---|---|---|---|
| Positive control and all treated groups with the extracts received injections of MIA Values expressed as Mean ± SME (standard mean error); I (%): percentage of inhibition. * p< 0.05, **p<0.01, ***p< 0,001 comparison with positive control (Mann Whitney U test) | | | | |

**Table 14. Effects of A. crispa and A. aculeata extracts on cartilage damage**

| Treatments | Changes of cartilage | | Extension of inflammation | | Cellular abnormalities | |
|---|---|---|---|---|---|---|
| | | I (%) | | I (%) | | I (%) |
| Negative control | 0** | | 0** | | 0** | |
| Positive control | 5.38±0.74 | | 3.63±0.52 | | 2.88±0.4 | |
| | | | | | | |
| *A. crispa* 100 mg/kg | 3.88±0.6** | 27.9 | 3.25±0.4** | 10.5 | 1.88±0.6* | 34.7 |
| *A. crispa* 200 mg/kg | 3.13±0.6*** | 41.8 | 2.88±0.4* | 20.7 | 1.38±0.5* | 52.1 |
| *A. crispa* 400 mg/kg | 2.75±0.46*** | 48.9 | 2.88±0.35* | 20.7 | 1.05±0.5*** | 63.6 |
| | | | | | | |
| *A. aculeata* 100 mg/kg | 3.85±0.6** | 28.4 | 3.25± 0.5** | 10.5 | 1.89±0.7* | 34.3 |
| *A. aculeata* 200 mg/kg | 3.12±0.7*** | 41.9 | 2.87±0.3* | 20.8 | 1.38±0.6** | 52.1 |
| *A. aculeata* 400 mg/kg | 2.74±0.5*** | 49.0 | 2.87±0.3* | 20.9 | 1.03±0.4*** | 64.2 |

| | | | | | | |
|---|---|---|---|---|---|---|
| Positive control and all treated groups with the extracts received injections of MIA Values expressed as Mean ± SME (standard mean error); I (%): percentage of inhibition. * p< 0.05, **p<0.01, ***p< 0.001 comparison with positive control (Mann Whitney U test) | | | | | | |

### Example 12

Several formulations were developed by using the active ingredient referred in the example 1, as follows: Solid oral form at doses between 50 and 1000 mg, like tablets containing corn starch, lactose, talc, gelatin, sodium croscarmellose, magnesium stearate, carboxymethylcellulose; or hard and soft gel capsules; Creams containing the active ingredient referred in the example 1 at concentrations between 0.1 and 90 %, and solid petrolatum, cetyl alcohol, polysorbate, propylene glycol, glycerin, stearyl alcohol, sodium lauryl sulfate, methylparaben, propylparaben, sodium phosphate monobasic, purified water, edetate disodium, carbopol, dimeticone, beeswax, and triethanolamine as excipients; Lotions and shampoos containing the active ingredient referred in the example 1 at concentrations between 0.1 and 90 %, and polysorbates, propylene glycol, carboxymethylcellulose, glycerin, sodium lauryl sulfate, methylparaben, propylparaben, sodium phosphate monobasic, purified water and triethanolamine as excipients.

## Claims

1. Active ingredient obtained from the fruits of both *Acrocomia crispa* and/or *Acrocomia aculeata* species, of the Arecaceae family, which contains a mixture of fatty acids from 6 to 28 carbon atoms (free or as acylglycerols or ethyl esters), and that also may contain sterols and high molecular weight fatty alcohols, and that mainly includes the following acids: caprilic (C_{8:0}), capric (C_{10:0}), lauric (C_{12:0}), myristic (C_{14:0}), palmitic (C_{16:0}), palmitoleic (C_{16:1}), stearic (C_{18:0}), oleic (C_{18:1}), linoleic (C_{18:2}) and linolenic (C_{18:3}) in the following proportion:
| Component | Relative Content (%) |
|---|---|
| caprilic acid (C_{8:0}) | < 3.0 |
| capric acid (C_{10:0}) | < 3.0 |
| lauric acid (C_{12:0}) | 30.0 - 40.0 |
| myristic acid (C_{14:0}) | 8.0 - 15.0 |
| palmitic acid (C_{16:0}) | 5.0 - 12.0 |
| palmitoleic acid (C_{16:1}) | < 3.0 |
| stearic acid (C_{18:0}) | 1.0 - 5.0 |
| oleic acid (C_{18:1})* | 20.0 - 35.0* |
| | |
|---|---|
| * Determined and reported as oleic acid, includes the mixture of oleic C_{18:1} (60 - 70 %); linoleic C_{18:2} (25 - 40 %) and linolenic C_{18:3} (< 10 %) acids. | |

2. Active ingredient as defined in claim 1, for use in the treatment and/or prevention of acute and chronic inflammation, of increased oxidative stress and of osteoarthritis.

3. Active ingredient as defined in claim 1, for use in the treatment and/or prevention of Benign Prostatic Hyperplasia and prostatitis.

4. Pharmaceutical composition **characterized by** containing 50 mg to 1000 mg of the active ingredient defined in claim 1.

5. Pharmaceutical composition according to claim 4, characterized to be formulated as solid oral form (hard capsule, softgel capsule, tablet), semisolid (suppository, cream, ointment), or liquid (emulsion, suspension, tincture, lotion or shampoo).

6. Pharmaceutical composition according to the claims 4 and 5,for use in the treating and/or prevent acute and chronic inflammation, of the increase of oxidative stress and osteoarthritis..

7. Pharmaceutical composition according to the claims 4 and 5, for use in the treating and/or prevent Benign Prostatic Hyperplasia and prostatitis.

8. Nutritional supplements **characterized by** containing 50 mg and 1000 mg of the active ingredient defined in claim 1.

9. Nutritional supplements according to the claim 8, **characterized by** its use in form of functional foods, nutraceuticals, dietetics, food additives, beverages and cosmetics.

10. Formulations of cosmetic function, **characterized by** containing the active ingredient as defined in claim 1 in concentrations between 0.1% and 90%.

11. Formulations with cosmetic function according to claim 10 administrated as creams, soaps, lotions, shampoos, bath gels, hair gels, ointments, body oils, lip protectors and skin care products, including sun screens.

## Patentansprüche

1. Wirkstoff erhalten aus den Früchten der beiden *Acrocomia crispa* und/oder *Acrocomia* aculeata-Arten der Arecaceae-Familie, welcher eine Mischung von Fettsäuren mit 6 bis 28 Kohlenstoffatomen (frei oder als Acylglycerole oder Ethylester) enthält, und welcher auch Sterole und Fettalkohole mit hohem Molekulargewicht enthalten kann, und welcher hauptsächlich die folgenden Säuren: Caprylsäure (C_{8:0}), Caprinsäure (C_{10:0}), Laurinsäure (C_{12:0}), Myristinsäure (C_{14:0}), Palmitinsäure (C_{16:0}), Palmitoleinsäure (C_{16:1}); Stearinsäure (C_{18:0}), Ölsäure (C_{18:1}), Linolsäure (C_{18:2}) und Linolensäure (C_{18:3}) in dem folgenden Verhältnis einschließt:
| Komponente | Relativer Gehalt (%) |
|---|---|
| Caprylsäure (C_{8:0}) | < 3,0 |
| Caprinsäure (C_{10:0}) | < 3,0 |
| Laurinsäure (C_{12:0}) | 30,0 - 40,0 |
| Myristinsäure (C_{14:0}) | 8,0 - 15,0 |
| Palmitinsäure (C_{16:0}) | 5,0 - 12,0 |
| Palmitoleinsäure (C_{16:1}) | < 3,0 |
| Stearinsäure (C_{18:0}) | 1,0 - 5,0 |
| Ölsäure (C_{18:1})* | 20,0 - 35,0* |
| | |
|---|---|
| * Bestimmt und angegeben als Ölsäure, schließt die Mischung von Ölsäure C_{18:1} (60 - 70 %); Linolsäure C_{18:2} (25 - 40 %) und Linolensäure C_{18:3} (< 10 %) ein. | |

2. Wirkstoff wie in Anspruch 1 definiert, zur Verwendung bei der Behandlung und/oder Verhütung einer akuten und chronischen Entzündung, von erhöhtem oxidativen Stress und von Osteoarthritis.

3. Wirkstoff wie in Anspruch 1 definiert, zur Verwendung bei der Behandlung und/oder Verhütung von gutartiger Prostatavergrößerung und Prostatitis.

4. Pharmazeutische Zusammensetzung, **dadurch gekennzeichnet, dass** sie 50 mg bis 1000 mg des in Anspruch 1 definierten Wirkstoffs enthält.

5. Pharmazeutische Zusammensetzung gemäß Anspruch 4, **dadurch gekennzeichnet, dass** sie als feste orale Form (Hartkapsel, Weichgelkapsel, Tablette), halbfeste Arzneiform (Zäpfchen, Creme, Salbe) oder Flüssigkeit (Emulsion, Suspension, Tinktur, Lotion oder Shampoo) formuliert ist.

6. Pharmazeutische Zusammensetzung gemäß den Ansprüchen 4 und 5, zur Verwendung bei der Behandlung und/oder Verhütung einer akuten und chronischen Entzündung, der Zunahme von oxidativem Stress und von Osteoarthritis.

7. Pharmazeutische Zusammensetzung gemäß den Ansprüchen 4 und 5, zur Verwendung bei der Behandlung und/oder Verhütung von gutartiger Prostatavergrößerung und Prostatitis.

8. Nahrungsergänzungsmittel, **dadurch gekennzeichnet, dass** sie 50 mg bis 1000 mg des in Anspruch 1 definierten Wirkstoffs enthalten.

9. Nahrungsergänzungsmittel gemäß Anspruch 8, **gekennzeichnet durch** ihre Verwendung in Form von funktionellen Lebensmitteln, Nutrazeutika, dietätischen Lebensmitteln, Nahrungsmittelzusatzstoffen, Getränken und Kosmetika.

10. Formulierungen mit kosmetischer Funktion, **dadurch gekennzeichnet, dass** sie den Wirkstoff wie in Anspruch 1 definiert in Konzentrationen zwischen 0,1 % und 90 % enthalten.

11. Formulierungen mit kosmetischer Funktion gemäß Anspruch 10 verabreicht als Cremes, Seifen, Lotionen, Shampoos, Badegele, Haargele, Salben, Körperöle, Lippenschutzmittel und Hautpflegeprodukte, einschließlich Sonnenschutzmittel.

## Revendications

1. Principe actif obtenu à partir des fruits des espèces *Acrocomia crispa* et/ou *Acrocomia aculeata,* de la famille des Arecaceae, qui contient un mélange d'acides gras de 6 à 28 atomes de carbone (libres ou en tant qu'acylglycérols ou qu'esters éthyliques), et qui peut également contenir des stérols et des alcools gras de poids moléculaire élevé, et qui comprend principalement les acides suivants : caprylique (C_{8:0}), caprique (C_{10:0}), laurique (C_{12:0}) , myristique (C_{14:0}), palmitique (C_{16:0}), palmitoléique (C_{16:1}), stéarique (C_{18:0}) , oléique (C_{18:1}), linoléique (C_{18:2}) et linolénique (C_{18:3}) dans la proportion suivants :
| Composant | Teneur relative (%) |
|---|---|
| acide caprylique (C_{8:0}) | < 3,0 |
| acide caprique (C_{10:0}) | < 3,0 |
| acide laurique (C_{12:0}) | 30,0 - 40,0 |
| acide myristique (C_{14:0}) | 8,0 - 15,0 |
| acide palmitique (C_{16:0}) | 5,0 - 12,0 |
| acide palmitoléique (C_{16:1}) | < 3,0 |
| acide stéarique (C_{18:0}) | 1,0 - 5,0 |
| acide oléique (C_{18:1})* | 20,0 - 35,0* |
| | |
|---|---|
| *Déterminé et indiqué en tant qu'acide oléique, mais comprend un mélange d'acides oléique C_{18:1} (60 - 70 %), linoléique C_{18:2} (25 - 40 %) et linolénique C_{18:3} (< 10 %). | |

2. Principe actif selon la revendication 1, pour une utilisation dans le traitement et/ou la prévention de l'inflammation aigüe et chronique, d'un stress oxydatif accru et de l'arthrose.

3. Principe actif selon la revendication 1, pour une utilisation dans le traitement et/ou la prévention d'une hyperplasie bénigne de la prostate et d'une prostatite.

4. Composition pharmaceutique **caractérisée en ce qu'**elle contient de 50 mg à 1 000 mg du principe actif selon la revendication 1.

5. Composition pharmaceutique selon la revendication 4, **caractérisée en ce qu'**elle est formulée sous une forme orale solide (gélule dure, gélule molle, comprimé), semi-solide (suppositoire, crème, pommade), ou liquide (émulsion, suspension, teinture, lotion ou shampoing).

6. Composition pharmaceutique selon les revendications 4 et 5, pour une utilisation dans le traitement et/ou la prévention de l'inflammation aigüe et chronique, d'un stress oxydatif accru et de l'arthrose.

7. Composition pharmaceutique selon les revendications 4 et 5, pour une utilisation dans le traitement et/ou la prévention d'une hyperplasie bénigne de la prostate et d'une prostatite.

8. Compléments nutritionnels **caractérisés en ce qu'**ils contiennent 50 mg et 1 000 mg du principe actif selon la revendication 1.

9. Compléments nutritionnels selon la revendication 8, **caractérisés par** leur utilisation sous la forme d'aliments fonctionnels, de produits nutraceutiques, de produits diététiques, d'additifs alimentaires, de boissons et de produits cosmétiques.

10. Formulations à fonction cosmétique, **caractérisées en ce qu'**elles contiennent le principe actif selon la revendication 1 dans des concentrations comprises entre 0,1 % et 90 % .

11. Formulations à fonction cosmétique selon la revendication 10, administrées sous la forme de crèmes, savons, lotions, shampoings, gels de bain, gels pour cheveux, pommades, huiles pour le corps, baumes à lèvres et produits de soins de la peau, y compris les écrans solaires.
